# FASCICULE DE BREVET EUROPEEN

(11) **EP 3 502 233 B1**
(45) Date de publication et mention de la délivrance du brevet: **25.11.2026**
(21) Numéro de dépôt: 18215652.1
(22) Date de dépôt: 21.12.2018
(51) Int. Cl.: C12M 3/00, C12M 1/00, C12N 5/073

(54) **SYSTEME D'ETUDE DU PASSAGE TRANSPLACENTAIRE EX-VIVO**
SYSTEM ZUR EX-VIVO-STUDIE DER TRANSPLAZENTAREN PASSAGE
SYSTEM FOR STUDYING EX-VIVO TRANSPLACENTAL TRANSFER

(30) Priorité: 21.12.2017 FR 1762820
(43) Date de publication de la demande: 26.06.2019
(73) Titulaire: Institut National de la Santé et de la Recherche Médicale (INSERM), 75013 Paris (FR); Université Paris Cité, 75006 Paris (FR); Fondation Cooperation Scientifique Dite Premup, 75006 Paris (FR); Université Paris-Saclay, 91190 Gif-sur-Yvette (FR)
(72) Inventeur: SIMASOTCHI, Christelle, 77680 ROISSY-EN-BRIE (FR); GIL, Sophie, 91430 IGNY (FR)
(74) Mandataire: Novagraaf Group

(56) Documents cités:
- WO-A2-02/064755
- R.A. DRAGOVIC ET AL: "Isolation of syncytiotrophoblast microvesicles and exosomes and their characterisation by multicolour flow cytometry and fluorescence Nanoparticle Tracking Analysis", METHODS, vol. 87, 3 April 2015 (2015-04-03), US, pages 64 - 74, XP055497887, ISSN: 1046-2023, DOI: 10.1016/j.ymeth.2015.03.028
- VALERO LUCIE ET AL: "Assessment of dually labelled PEGylated liposomes transplacental passage and placental penetration using a combination of twoex-vivohuman models: the dually perfused placenta and the suspended villous explants", INTERNATIONAL JOURNAL OF PHARMACEUTICS, vol. 532, no. 2, 27 July 2017 (2017-07-27), pages 729 - 737, XP085239833, ISSN: 0378-5173, DOI: 10.1016/J.IJPHARM.2017.07.076
- SCHNEIDER H ET AL: "Dual in vitro perfusion of an isolated lobe of human placenta: method and instrumentation", vol. 13, 1 January 1985 (1985-01-01), pages 40 - 47, XP009508804, ISSN: 0304-4246, Retrieved from the Internet <URL:https://www.karger.com/Article/Abstract/410668>
- MORTIMER R H ET AL: "Secretion and transfer of the thyroid hormone binding protein transthyretin by human placenta", PLACENTA, W.B. SAUNDERS, GB, vol. 33, no. 4, 3 January 2012 (2012-01-03), pages 252 - 256, XP028460586, ISSN: 0143-4004, [retrieved on 20120107], DOI: 10.1016/J.PLACENTA.2012.01.006
- SIGRID CONINGS ET AL: "Integration and validation of the ex vivo human placenta perfusion model", JOURNAL OF PHARMACOLOGICAL AND TOXICOLOGICAL METHODS., vol. 88, 1 November 2017 (2017-11-01), US, pages 25 - 31, XP055516256, ISSN: 1056-8719, DOI: 10.1016/j.vascn.2017.05.002
- STEFANIE GRAFMÜLLER ET AL: "Determination of the Transport Rate of Xenobiotics and Nanomaterials Across the Placenta using the ex vivo Human Placental Perfusion Model", JOURNAL OF VISUALIZED EXPERIMENTS, no. 76, 18 June 2013 (2013-06-18), XP055516386, DOI: 10.3791/50401
- JENNIFER SOUTHCOMBE ET AL: "The Immunomodulatory Role of Syncytiotrophoblast Microvesicles", PLOS ONE, vol. 6, no. 5, 25 May 2011 (2011-05-25), pages e20245, XP055516565, DOI: 10.1371/journal.pone.0020245
- CIERNA ZUZANA ET AL: "Intermediate trophoblast-A distinctive, unique and often unrecognized population of trophoblastic cells", ANNALS OF ANATOMY, JENA, DE, vol. 204, 12 November 2015 (2015-11-12), pages 45 - 50, XP029423858, ISSN: 0940-9602, DOI: 10.1016/J.AANAT.2015.10.003

## Description

### DOMAINE DE L'INVENTION

La présente invention concerne un dispositif adapté pour l'étude des échanges transplacentaires entre le fœtus et la mère et plus particulièrement des échanges au niveau d'un placenta dont la structure est non consolidée, le placenta étant préférentiellement âgé d'un mois et demi à quatre mois. L'invention concerne également une méthode d'étude du passage transplacentaire de molécules, en particulier des xénobiotiques mais également de molécules endogènes tels que les nutriments, depuis la circulation maternelle vers la circulation fœtale ou bien depuis la circulation fœtale vers la circulation maternelle.

### ETAT DE LA TECHNIQUE

Le placenta est l'organe à l'interface entre la mère et le fœtus. Il est indispensable à l'implantation de l'embryon puis au maintien de la grossesse et au bon développement du fœtus.

La détermination du passage transplacentaire des médicaments et autres xénobiotiques représente un domaine de recherche primordial pour des questions évidentes de santé publique.

Le modèle du placenta humain à terme perfusé *ex-vivo* représente à ce jour la seule méthode dynamique restituant les conditions physiologiques de fonctionnement *in utero* du placenta humain en fin de grossesse. Cette méthodologie est bien maîtrisée mais ne concerne que les placentas obtenus après la délivrance (placentas dits « à terme »).

A cet égard, Schneider *et al.* (Schneider, H., Panigel, M., & Dancis, J. (1972), Transfer across the perfused human placenta of antipyrine, sodium, and leucine, American journal of obstetrics and gynecology, 114(6), 822-828) décrivent une méthode pour la perfusion *ex-vivo* d'antipyrine, de sodium et de L-leucine dans un cotylédon isolé. Schneider et Husch (Schneider, H., Huch, A. (1985), Dual in vitro perfusion of an isolated lobe of human placenta: method and instrumentation. Contrib Gynecol Obstet., 13:40-47) décrivent également une méthode et un dispositif de perfusion de cotylédon isolé. De même, Valero *et al.* (Valero, L., Alhareth, K., Gil, S. et al. (2017), Assessment of dually labelled PEGylated liposomes transplacental passage and placental penetration using a combination of two ex-vivo human models: the dually perfused placenta and the suspended villous explants. Int J Pharm., 532(2):729-737) utilisent un système équivalent de double perfusion de placenta à terme pour étudier le passage transplacentaire de liposomes. Dragovic *et al.* (Dragovic, R.A., Collett, G.P., Hole, P. et al., (2015) Isolation of syncytiotrophoblast microvesicles and exosomes and their characterisation by multicolour flow cytometry and fluorescence Nanoparticle Tracking Analysis. Methods, 87:64-74) utilisent un système de perfusion de lobe de placenta à terme pour produire des vésicules extracellulaires de syncytiotrophoblastes (STBEV). Egalement, Conings et al. (J. of Pharmacological and Toxicological Methods, 88 (2017) 25-31) décrivent un protocole de perfusion *ex-vivo* d'un placenta humain à terme pour l'étude du passage transplacentaire de médicaments ou de composés environnementaux. Conings *et al.* décrivent ainsi un logement muni d'orifices permettant d'introduire des cathéters pour la circulation fœtale et d'une sortie fluidique pour le flux maternel. Le placenta post-partum est récupéré, les cotylédons intacts identifiés, les veines et artères fœtales d'un cotylédon intact sont identifiées et canulées afin de procéder à un lavage avec un milieu adéquat. Du côté maternel, la circulation maternelle est établie via un cathéter planté dans l'espace intervilleux du cotylédon et relié à une pompe. Le placenta est ainsi disposé dans le logement, face maternelle vers le haut, dans laquelle le cathéter est planté, face fœtale vers le bas, artère et veine connectées à des cathéters qui sont eux même reliés à une pompe péristaltique afin d'établir la circulation fœtale.

Ces dispositifs et les méthodes mises en œuvre grâce à celui-ci sont adaptés pour un placenta à terme, dont l'unité fonctionnelle est le cotylédon et dont la structure est bien identifiée et dite consolidée. Cette structure est par exemple décrite par Boyd, J.D. et Hamilton, W. J.( Boyd, J. D., & Hamilton, W. J., 1970, The human placenta, Vol. 212, Heffer). Cette structure du placenta permet de perfuser l'espace intervilleux avec la solution à tester par l'insertion directe du cathéter dans le cotylédon au niveau de la face maternelle à travers la décidue.

Cependant, un tel dispositif n'est absolument pas adapté pour l'étude et l'utilisation d'un placenta dont la structure est non consolidée.

Le développement du placenta humain au cours de la grossesse et particulièrement au cours des quatre premiers mois de grossesse est tout à fait particulier, spécifique de l'espèce humaine et primordial pour le développement du fœtus et l'issue de la grossesse.

Ainsi, après la fécondation, au moment de la placentation, l'embryon s'entoure d'une couche de trophoblastes où s'organisent une arborescence de villosités et la circulation utéro-placentaire.

Jusqu'à la fin du premier trimestre, les artères maternelles sont obstruées par des trophoblastes et les échanges se font avec le plasma. C'est après le premier trimestre que les bouchons trophoblastiques commencent à disparaître, laissant ainsi le sang irriguer l'espace intervilleux, l'acquisition d'une structure bien consolidée se mettant définitivement en place au cours du cinquième mois de grossesse (Boyd, J. D., & Hamilton, W. J., 1970, The human placenta, Vol. 212, Heffer).

La villosité placentaire est formée d'un axe mésenchymateux d'origine embryonnaire où se développent les vaisseaux fœtaux et est bordée par le trophoblaste. Les cellules trophoblastiques villeuses forment une couche de cellules mononucléées (cytotrophoblastes) qui se différencient par fusion en syncytiotrophoblastes multinucléés recouvrant l'ensemble des villosités. Le syncytiotrophoblaste se forme et se régénère tout au long de la grossesse par fusion et différenciation des cellules cytotrophoblastiques sous-jacentes. Ce syncytiotrophoblaste se caractérise à sa surface apicale par une membrane microvillositaire qui, en contact avec le sang maternel, assure les fonctions hormonales, d'échanges et d'homéostasie entre la mère et le fœtus.

Ainsi, l'épaisseur de l'interface materno-fœtale séparant les circulations maternelle et fœtale diminue au cours de la grossesse. Au cours du premier trimestre, les cytotrophoblastes forment une couche continue. Au cours de la grossesse, cette couche devient plus fine, présentant des cellules isolées mais jointives situées entre le syncytiotrophoblaste et la membrane basale. L'épaisseur de la barrière placentaire diminue donc de sensiblement 100 µm lors du deuxième mois de grossesse à sensiblement 5 µm en fin de grossesse. De même, on observe une augmentation progressive de la surface d'échange en raison de la croissance du placenta, concomitante de la poursuite du processus de ramification de l'arbre villositaire. Ainsi, la surface d'échange évolue de sensiblement 3 m² dans un placenta âgé de 28 semaines d'aménorrhée à sensiblement 15 m² dans un placenta à terme. Enfin, le débit utérin, estimé à environ 50 mL.min⁻¹ dans un placenta âgé de 10 semaines d'aménorrhée va progressivement augmenter pour atteindre 400-500 mL.min⁻¹ dans un placenta à terme. Ainsi, les mécanismes d'échanges entre la mère et le fœtus apparaissent très différents entre un placenta âgé de 1 à 4 mois d'un placenta à terme, du fait des différences d'épaisseur de la barrière placentaire, d'une consolidation ou non de la structure du placenta et des différences de surface et de circulation entre le début de la grossesse et le terme.

Il est connu que l'exposition d'une femme enceinte à des agents xénobiotiques (par exemple des médicaments, polluants, cosmétiques et additifs alimentaires) requiert une grande vigilance. En effet, ces différentes molécules, une fois dans la circulation sanguine maternelle sont susceptibles d'agir directement sur le fœtus, si elles traversent la barrière placentaire.

Le passage des molécules à travers les membranes placentaires s'effectue grâce à différents mécanismes que sont le transport passif, le transport facilité et le transport actif.

Le transport passif peut être décrit par la loi de Fick : ${V}_{diff} = D . S . \frac{{C}_{m} - {C}_{f}}{A} ,$ où V_{diff} est le taux de diffusion d'une molécule, D le coefficient de diffusion d'une molécule, S l'aire de surface d'échange, Cₘ la concentration dans la circulation maternelle, C_{f} la concentration dans la circulation fœtale et A l'épaisseur de la membrane.

Le transport passif n'est pas saturable et n'est pas sujet à l'inhibition compétitive. Quand les produits traversent le placenta de façon passive, leur passage est fonction de leur concentration dans la circulation maternelle, de leurs propriétés physico-chimiques et des propriétés du placenta. Des variations de ces paramètres peuvent modifier le passage des médicaments.

Le transport par diffusion passive est favorable aux molécules ayant un faible poids moléculaire (<500 Da), très liposolubles et non ionisées.

Les caractéristiques physicochimiques d'une molécule ne suffisent pas à prédire un passage ou un non passage de la molécule. Le placenta n'est pas qu'une membrane passive. En effet, des transporteurs sont connus dans le placenta. Ces transporteurs sont principalement localisés dans la membrane microvillositaire et/ou dans la membrane basale du syncytiotrophoblaste. Le cytotrophoblaste et l'endothélium des capillaires fœtaux expriment également des transporteurs. Ainsi, des différences d'expression de transporteurs entre cytotrophoblaste et syncytiotrophoblaste pourraient être à l'origine d'une distribution différente d'une molécule entre le compartiment maternel et le compartiment fœtal tout au long de la grossesse. Certains transporteurs dits transporteurs d'influx (permettant un transport facilité et transport actif secondaire, couplé à un antiport par exemple), facilitent le transfert de molécules exogènes vers le fœtus. D'autres transporteurs, dits transporteurs d'efflux (permettant un transport actif primaire et étant ATP dépendant), limitent ce transfert.

En outre, les profils d'expression des transporteurs varient au cours de la grossesse. Ainsi, Berveiller *et al.* (Berveiller, P., Degrelle, S. A., Segond, N., Cohen, H., Evain-Brion, D., & Gil, S., 2015, Drug transporter expression during in vitro differentiation of first-trimester and term human villous trophoblasts. Placenta, 36(1), 93-96) décrivent des profils différents d'expression des transporteurs pour un placenta du premier trimestre et pour un placenta à terme.

Ces différences d'expression des transporteurs se surajoutent aux différences structurelles préalablement décrites entre un placenta âgé de un mois et demi à 4 mois et un placenta à terme.

Aussi, grâce aux modèles actuels (tel que ceux décrits par Conings *et* al.) et bien que des connaissances existent en ce qui concerne le passage transplacentaire de molécules médicamenteuses entre la circulation maternelle et la circulation fœtale en fin de grossesse, on ne dispose que de très peu d'éléments sur la nature qualitative et quantitative des échanges materno-fœtaux et le passage transplacentaire en début de grossesse, qui ne permettent pas de prédire le passage transplacentaire des molécules.

Une telle connaissance concernant le début de la grossesse est d'autant plus importante que c'est durant le premier trimestre de grossesse que les risques sont majeurs pour le fœtus. En effet, cette période est notamment le siège de l'organogenèse et est donc déterminante pour l'apparition de malformations. Aussi, il est admis que des pathologies comme la prééclampsie s'initient dès le premier trimestre de grossesse (des marqueurs prédictifs étant détectés entre 11 et 13 semaines d'aménorrhée).

### RESUME DE L'INVENTION

Un but de l'invention est de proposer un système et une méthode permettant de déterminer le passage (ou le non passage) transplacentaire d'un composé test et ce dès le premier trimestre de grossesse. Un autre but de l'invention est de proposer un modèle pour étudier certaines pathologies d'origine placentaire en début de grossesse.

Les inventeurs ont développé un système adapté à la perfusion *ex-vivo* d'un placenta de début de grossesse qui ne présente pas de structure consolidée et notamment pas de septa intercotylédonaires définissant des cotylédons. Le système et la méthode proposés permettent de rétablir, par exemple dans un placenta âgé d'environ 8 à 19 semaines d'aménorrhée (SA), une double circulation materno-fœtale dans le placenta, préférentiellement en contrôlant des paramètres physiologiques prédéterminés.

L'intérêt est majeur, notamment pour tous les questionnements autour de la prise médicamenteuse en début de grossesse et de la connaissance d'un passage des molécules dans la circulation fœtale. En effet, l'exposition aux xénobiotiques pose essentiellement un problème dans cette période d'intérêt du début de grossesse, et notamment les 4 premier mois.

Un objet de l'invention est un système d'étude du passage ou de la métabolisation transplacentaire *ex-vivo* d'un composé test, tel que décrit à la revendication 1.

Le placenta est préférentiellement un placenta de grand singe, et encore plus préférentiellement un placenta humain issu d'une interruption volontaire de grossesse (IVG) ou d'une interruption médicale de grossesse, considéré dans de tels cas comme un déchet biologique.

Dans un mode de réalisation particulier, le diamètre de l'orifice est inférieur à 5 cm et d'au moins 1 cm.

Dans un mode de réalisation particulier, le système comprend une première conduite, le cathéter d'injection et le cathéter de collecte étant reliés fluidiquement par au moins la première conduite, apte à rendre l'écoulement de la solution foetale f en circuit fermé, le système comprenant une pompe adaptée pour entraîner un écoulement dans ladite première conduite.

Dans un mode de réalisation particulier, le système comprend une deuxième conduite reliée à la chambre de perfusion, l'entrée fluidique et la sortie fluidique étant reliées fluidiquement par au moins la deuxième conduite, apte à rendre l'écoulement de la solution maternelle m en circuit fermé, le système comprenant une pompe adaptée pour entraîner un écoulement dans la deuxième conduite.

Dans un mode de réalisation particulier, le système comprend un placenta ne présentant pas de septa intercotylédonaires définissant des cotylédons et qui, quand il est d'origine humaine, est un déchet biologique issu d'une interruption volontaire de grossesse ou d'une interruption médicale de grossesse.

Dans un mode de réalisation particulier, le système comprend un élément choisi parmi un capteur de pH, un capteur de température, un capteur d'oxygène, un capteur de pression, un capteur de débit de la solution maternelle et/ou de la solution fœtale et un moyen de mesure de la concentration du composé test et/ou d'un composé métabolite dont le passage transplacentaire est testé.

Dans un mode de réalisation particulier, le système comprend des moyens d'asservissement de la concentration d'un composé de la solution maternelle et/ou de la solution fœtale à une valeur consignée.

Dans un mode de réalisation particulier, le système comprend des moyens d'asservissement du débit de la solution maternelle et/ou de la solution fœtale à une valeur consignée.

Dans un mode de réalisation particulier, le système comprend plusieurs parois interchangeables avec des orifices de tailles différentes, centrés ou décentrés, de manière à adapter au mieux le système au placenta étudié.

Dans un mode de réalisation particulier, la cavité présente une ouverture configurée pour permettre un prélèvement de la solution maternelle.

Un autre objet de l'invention est une méthode *ex-vivo* d'étude du passage transplacentaire d'au moins un composé test ou de la métabolisation d'un composé test en un composé métabolite telle que décrite à la revendication 10.

Dans un mode de réalisation particulier, la méthode est mise en œuvre par un système de l'invention décrit précédemment.

Dans un mode de réalisation particulier, l'étape d) de mise en contact est réalisée sans cathéter.

Dans un mode de réalisation particulier, l'étape d) de mise en contact est réalisé par trempage desdites villosités dans la solution maternelle.

Dans un mode de réalisation particulier, la méthode comprend une étape b') après l'étape b) et avant l'étape c), l'étape b') comprenant la perfusion du placenta par injection via un cathéter au niveau de l'artère fœtale d'une solution fœtale comprenant un indicateur coloré permettant d'identifier au moins une villosité placentaire irriguée par ladite solution fœtale.

Dans un mode de réalisation particulier, la méthode est caractérisée en ce que la solution fœtale et/ou la solution maternelle contient un composé marqueur, ledit composé marqueur présentant un taux de passage transplacentaire prédéterminé.

Dans un mode de réalisation particulier, le composé marqueur est l'antipyrine.

Dans un mode de réalisation particulier, on normalise la quantité de composé test et/ou la quantité de composé métabolite détectée lors de l'étape e) par une quantité de composé marqueur mesurée.

Dans un mode de réalisation particulier, la solution maternelle comprend un inhibiteur de transporteur placentaire.

Dans un mode de réalisation particulier, la cavité remplie de solution maternelle est alimentée en ladite solution par une entrée fluidique et vidée en ladite solution par une sortie fluidique afin de créer un écoulement de solution maternelle à un débit compris entre 1 et 20 mL.min⁻¹, de préférence entre 1 et 10 mL.min⁻¹, de manière encore plus préférée entre 5 et 10 mL.min⁻¹.

Dans un mode de réalisation particulier, le débit de solution fœtale est compris entre 10 µL.min⁻¹ et 300 µL.min⁻¹, de préférence entre 50 µL.min⁻¹ et 300 µL.min⁻¹.

Dans un mode de réalisation particulier, la méthode est mise en œuvre dans des conditions mimant au moins une caractéristique d'une pathologie de la grossesse.

Dans un mode de réalisation particulier, la solution maternelle et/ou la solution fœtale comprend un microorganisme ou un fragment de microorganisme choisi au moins parmi une bactérie, un virus, un organisme unicellulaire et une molécule issue d'une bactérie, d'un virus ou d'un organisme unicellulaire.

### PRESENTATION DES DESSINS

D'autres caractéristiques et avantages ressortiront encore de la description qui suit, laquelle est purement illustrative et non limitative, et doit être lue en regard des figures annexées, parmi lesquelles :
- la figure 1 illustre schématiquement une partie d'un système d'étude du passage ou de la métabolisation transplacentaire *ex-vivo* d'un composé test,
- la figure 2 illustre une vue de côté d'une partie du système,
- la figure 3 illustre une vue de côté d'une partie du système,
- la figure 4 illustre une vue de dessus d'une partie du système,
- la figure 5 illustre une coupe d'une partie du système,
- la figure 6 est une photographie du système,
- la figure 7 illustre une méthode d'étude du passage d'un composé test,
- la figure 8 est une photographie illustrant la cathétérisation de la face fœtale d'un placenta dont la structure est non consolidée,
- la figure 9 est une photographie illustrant l'injection d'un indicateur coloré dans le placenta dont la structure est non consolidée, et
- la figure 10 est un diagramme illustrant la cinétique du taux de transfert de l'antipyrine dans un placenta.

### DEFINITIONS

On désigne par le terme « diamètre » la longueur maximale d'un segment passant par le barycentre d'un élément.

On désigne par le terme « composé test » un xénobiotique (*i*.*e*. composé étranger à l'organisme) mais également une molécule endogène tels qu'un nutriment, hormone ou tout autre molécule biologique endogène ou encore une molécule biologique exogène. Plus particulièrement « composé test » peut comprendre un microorganisme ou un fragment de microorganisme choisi parmi au moins une bactérie, un virus, un organisme unicellulaire et une molécule issue d'une bactérie, d'un virus ou d'un organisme unicellulaire. « Composé test » peut également comprendre un mélange d'au moins deux composés tels que mentionnés ci-dessus.

On désigne par le terme « métabolisation placentaire » la transformation biochimique d'une substance, d'un composé lors de son passage par la structure placentaire, et notamment lors des échanges materno-fœtaux. Ainsi un « composé métabolite » est au moins une des molécules résultant de cette transformation biochimique.

On désigne par le terme « taux de transfert » d'un composé le rapport de la concentration fœtale veineuse de ce composé, c'est-à-dire la concentration de ce composé dans une circulation fluidique correspondant à la circulation fœtale, sur la concentration maternelle de ce composé, c'est-à-dire la concentration de ce composé dans la circulation fluidique correspondant à la circulation maternelle. Le taux de transfert peut par exemple être exprimé en pourcents.

On désigne par le terme « placenta dont la structure est non consolidée » un placenta qui ne présente pas de septa intercotylédonaires définissant des cotylédons. La structure consolidée se mettant en place au cours du 5^{ème} mois, un placenta dont la structure est non consolidée est un placenta de moins de 5 mois de grossesse. Ledit placenta est un placenta d'un primate de grande taille et de préférence un placenta humain. En particulier, un placenta dont la structure n'est pas consolidée ne comprend pas de cotylédon. De manière générale, le ou les placentas de l'invention sont âgés d'un mois à 5 mois et préférentiellement d'un mois et demi à 4 mois.

On désigne par le terme « pathologie de la grossesse » les pathologies dont l'étiologie est en lien avec la grossesse, touchant soit la mère soit le fœtus soit les deux, comme par exemple la prééclampsie ou des infections représentant un risque particulier lors de la gestation.

On désigne par « grand singes » les orang-outans, les gorilles, les chimpanzés et les hommes.

### DESCRIPTION DETAILLEE DE L'INVENTION

### Architecture générale du système

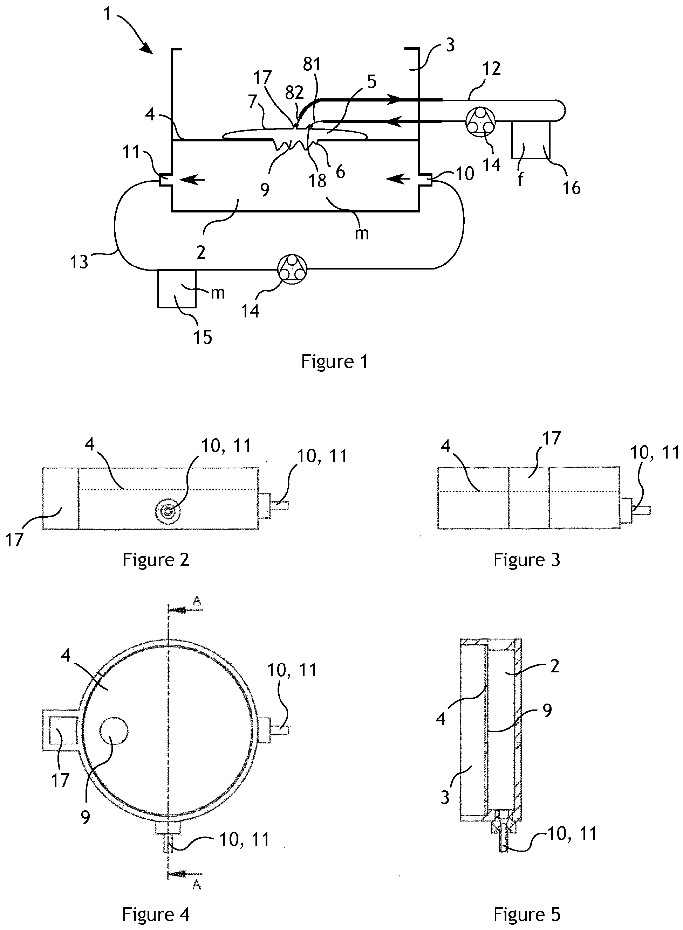

En référence à la figure 1, le système 1 comprend une chambre de réception 3 d'un placenta 5 dont la structure est non consolidée, de préférence âgé d'un mois et demi à 4 mois. La chambre de réception 3 est partiellement définie par une paroi 4, sur laquelle est déposée le placenta 5. La paroi 4 présente un orifice 9. Le système 1 comprend également une cavité 2, partiellement définie par la paroi 4. Ainsi, la paroi 4 sépare la cavité 2 et la chambre de réception 3.

Un placenta 5 est déposé sur l'orifice 9. Le placenta 5 présente deux faces distinctes : une face maternelle 6 et une face fœtale 7.

Le placenta 5 est agencé de manière à présenter la face fœtale 7 vers la partie supérieure du système, qui se situe au-dessus de la paroi 4 et de la face maternelle 6. La face fœtale 7 présente des vaisseaux fœtaux, et préférentiellement une artère fœtale et une veine fœtale. L'artère fœtale et la veine fœtale peuvent être distinguées ou détectables par un opérateur par exemple après 8 semaines d'aménorrhée, préférentiellement après 10 semaines d'aménorrhée. Un cathéter d'injection 81 est relié fluidiquement à un vaisseau fœtal, préférentiellement à l'artère fœtale. Un cathéter de collecte 82 est relié fluidiquement à un vaisseau fœtal, préférentiellement à une veine fœtale. Un cathéter 8 (comme un cathéter d'injection 81 et/ou un cathéter de collecte 82) peut par exemple comprendre un tube en peroxyde de silicone et un tube ou une aiguille capillaire rigide, par exemple métallique, dont une extrémité est introduite à l'intérieur du tube en peroxyde de silicone. Le tube en peroxyde de silicone et le capillaire métallique peuvent par exemple être scellés par de la colle cyanoacrylate. Ainsi, l'autre extrémité du capillaire rigide peut être introduite dans un vaisseau fœtal et en particulier dans une veine fœtale ou dans une artère fœtale d'un placenta 5. Les différents cathéters 8 peuvent être supportés à l'intérieur de la chambre de réception 3 par un support présentant une forme de rigole, les cathéters 8 étant agencés dans la rigole.

Le cathéter d'injection 81 est adapté pour injecter une solution fœtale f dans un vaisseau fœtal et préférentiellement dans une artère fœtale, de manière à irriguer des villosités placentaires avec la solution fœtale f.

Le cathéter de collecte 82 est adapté pour collecter la solution fœtale f depuis un vaisseau fœtal et préférentiellement depuis la veine fœtale.

Un réservoir de solution fœtale 16 peut être relié fluidiquement au cathéter d'injection 81 par une tubulure. L'injection de la solution fœtale f dans le placenta 5 peut être contrôlée par une pompe 14, par exemple une pompe péristaltique contrôlant le débit de solution fœtale f dans la tubulure reliant le réservoir de solution fœtale 16 et le cathéter d'injection 81.

Dans un mode de réalisation particulier, l'écoulement de solution fœtale f peut être en circuit fermé. Une première conduite 12 relie au moins indirectement le cathéter d'injection 81 et le cathéter de collecte 82. Ainsi, il est possible de mimer la circulation fœtale en circuit fermé.

La solution fœtale f peut par exemple comprendre un tampon adapté à la culture de cellules dans un environnement comprenant du CO₂. La solution fœtale f peut comprendre un ou plusieurs composés test C, dont on souhaite étudier le passage du fœtus vers la mère et/ou la métabolisation transplacentaire. La solution fœtale f peut par exemple comprendre un tampon de type DPBS, EBSS, HBSS ou PBS. La solution peut être une solution adaptée à la conservation d'organes (par exemple décrite dans le brevet EP1164841B9), ou du sang (sang naturel, par exemple du sang du patient dont est issu le placenta 5, ou sang artificiel). La solution fœtale f peut également comprendre du glucose, de la L-glutamine, de la pénicilline, de la streptomycine, de la BSA ou de la HSA (*Bovine Serum Albumin* ou *Human Serum Albumin*) et/ou de l'héparine. Préférentiellement, la concentration en BSA de la solution fœtale f est comprise entre 1 g.L⁻¹ et 20 g.L⁻¹, et préférentiellement entre 5 g.L⁻¹ et 10 g.L⁻¹. Préférentiellement, dans le cas où le glucose n'est pas un composé pour lequel la perméabilité transplacentaire est testée, la concentration en glucose peut être sensiblement égale à 1g.L⁻¹.

Le placenta 5 est agencé de manière à présenter la face maternelle 6 en regard de la paroi 4. La face maternelle 6 du placenta 5 présente des villosités placentaires. Ainsi, au moins une partie des villosités placentaires sont présentées à la cavité 2 par l'orifice 9. Préférentiellement, l'ensemble des villosités placentaires irriguées par la solution fœtale sont mises en contact avec la solution maternelle m. Dans un mode de réalisation particulier, le placenta 5 est un placenta âgé d'un mois et demi à 4 mois de grossesse (soit 8 à 19 semaines d'aménorrhée). Ledit placenta, quand il est d'origine humaine, étant un déchet biologique issu d'une interruption volontaire de grossesse ou d'une interruption médicale de grossesse.

La cavité 2 comprend une solution maternelle m. La solution maternelle m peut comprendre un ou plusieurs composés test C, dont on souhaite étudier le passage et/ou la métabolisation transplacentaire. Ainsi, une partie de la face maternelle 6 du placenta 5 est en contact avec la solution maternelle m. Les inventeurs ont découvert qu'il était possible d'éviter une cathétérisation de la face maternelle 6, impossible en raison de la morphologie du placenta 5 dont la structure est non consolidée, en remplaçant une perfusion mise en œuvre par un ou plusieurs cathéters connectés à la face maternelle 6 par des moyens de mise en contact d'au moins une partie des villosités placentaires, ou d'au moins une partie de la surface des villosités placentaires de la face maternelle 6 du placenta 5 avec la solution maternelle m. En outre, les villosités du placenta ou les villosités irriguées par la solution fœtale, qui ne sont pas recouvertes par la caduque puisque le placenta est un placenta non consolidé, trempent librement dans la solution maternelle, ce qui est particulièrement avantageux car proche des conditions physiologiques et ne nécessite pas, comme mentionné, de léser le placenta du côté maternel en y plantant un ou plusieurs cathéters. Il est ainsi possible d'étudier le passage ou la métabolisation transplacentaire d'un composé test C sur un placenta 5 âgé d'un mois et demi à quatre mois dont la structure est non consolidée.

Dans un mode de réalisation particulier, la cavité 2 est une chambre de perfusion comprenant une entrée fluidique 10 et une sortie fluidique 11. L'entrée fluidique 10 et la sortie fluidique 11 permettent de commander un écoulement de solution maternelle m dans la chambre de perfusion ; cet écoulement est illustré par des flèches dans la cavité 2 dans la figure 1.

Un réservoir 15 de solution maternelle m comprend la solution maternelle m. Ce réservoir 15 de solution maternelle m est relié fluidiquement à l'entrée fluidique 10.

Dans un mode de réalisation particulier, l'écoulement de solution maternelle m peut être en circuit fermé. Une deuxième conduite 13 relie au moins indirectement l'entrée fluidique 10 et la sortie fluidique 11. Ainsi, il est possible de mimer la circulation maternelle en circuit fermé.

La solution maternelle m peut par exemple comprendre un tampon adapté à la culture de cellules dans un environnement comprenant du CO₂. La solution maternelle m peut par exemple comprendre un tampon de type DPBS, EBSS, HBSS ou PBS. La solution peut être une solution adaptée à la conservation d'organes (par exemple décrite dans le brevet EP1164841 B9), ou du sang (sang naturel, par exemple du sang du patient dont est issu le placenta, ou sang artificiel). La solution maternelle m peut également comprendre au moins un élément choisi parmi du sang de patient pré-éclamptique, impaludé, drépanocytaire, ou plus généralement du sang comprenant un agent pathogène. La solution maternelle peut également comprendre du glucose, de la L-glutamine, de la pénicilline, de la streptomycine, de la BSA ou de la HSA et/ou de l'héparine. Préférentiellement, la concentration en BSA ou HSA de la solution fœtale est comprise entre 1 g.L⁻¹ et 20 g.L⁻¹, et préférentiellement entre 5 g.L⁻¹ et 10 g.L⁻¹. Préférentiellement, dans les cas où le glucose n'est pas un composé pour lequel la perméabilité transplacentaire est testée, la concentration en glucose est de 1 g.L⁻¹.

Dans un mode de réalisation particulier, on impose un pH différent d'une part dans la solution maternelle m et d'autre part dans la solution fœtale f. Le pH imposé dans la solution maternelle m peut être sensiblement égal à 7,4. Le pH imposé dans la solution fœtale f peut être sensiblement égal à 7,2. Ainsi, il est possible de mimer la différence physiologique de pH entre le pH du sang fœtal et le pH du sang maternel.

Dans un mode de réalisation encore plus particulier, le pH imposé dans la solution maternelle m est inférieur à 7.4, voire compris entre 7.4 et 7.2. Ceci est particulièrement intéressant pour mimer l'acidose maternelle. Dans un mode de réalisation particulier, le pH imposé dans la solution maternelle est supérieur à 7.42, ceci est particulièrement intéressant pour mimer l'alcalose métabolique maternelle. Dans un mode de réalisation particulier, le pH de la solution fœtale est inférieur à 7,2. Ainsi, il est possible de mimer l'acidose fœtale.

Les figures 2, 3, 4 et 5 illustrent respectivement une vue de côté, une vue d'un autre côté, une vue de dessus et une vue de coupe de la même partie du système 1.

En référence aux figures 2, 3, 4 et 5, l'orifice 9 peut être de forme circulaire. De préférence, le diamètre de l'orifice 9 peut être inférieur à la taille du placenta 5 de manière à ce que celui-ci soit supporté par la paroi 4. De manière encore plus préférée, le diamètre de l'orifice 9 peut être inférieur ou égal à 5 cm. De manière préférée, le diamètre de l'orifice 9 est au moins de 1 cm. Ainsi, la face maternelle 6 du placenta 5 peut être mise en contact avec la solution maternelle m tout en permettant au placenta 5 d'être soutenu ou maintenu par le reste de la paroi 4 dans la chambre de réception 3. De plus, ce diamètre permet de mettre en contact la solution maternelle m avec une partie des villosités placentaires, c'est-à-dire une partie de la surface de la face maternelle 6 présentant des villosités placentaires correspondant aux villosités placentaires irriguées par la cathétérisation de la face fœtale 7, de préférence toutes les villosités placentaires irriguées par la cathétérisation de la face fœtale 7.

L'orifice 9 peut également être décentré par rapport à la paroi 4. Ainsi, il est possible d'adapter le système à la configuration, la forme et la taille du placenta 5 ou à la localisation des cathéters 8 reliés au placenta. La localisation des cathéters 8 dépend de la localisation des vaisseaux fœtaux isolés pour l'étude. Les manipulations du placenta 5 peuvent ainsi être limitées, de manière à éviter d'endommager le placenta 5.

De manière préférée, le système selon l'invention comprend une cavité 2 et une chambre de réception 3, et plusieurs parois 4 interchangeables dans le système comprenant des orifices 9 de tailles différentes, centrés ou décentrés, de manière à adapter au mieux le système au placenta étudié.

Dans un mode de réalisation particulier la paroi 4 contient des moyens de maintien des cathéters fœtaux, par exemple sous la forme d'une rigole qui dirige les cathéters et/ou la tubulure vers des orifices ménagés dans la paroi de la chambre de réception 3 et/ou du capot.

Le système 1 peut également comprendre une ouverture 17 configurée pour permettre un prélèvement de la solution maternelle m. Un prélèvement de la solution maternelle m peut par exemple permettre d'analyser la composition de la solution maternelle m à différents temps de l'étude, et ainsi d'évaluer la cinétique de passage ou de métabolisation d'un composé test C. La cavité 2 peut également comprendre une ouverture munie d'une vanne ou d'un robinet, permettant de prélever la solution maternelle m.

Le système 1 peut comprendre un ou plusieurs capteurs intégrés, permettant d'éviter de réaliser des prélèvements lors de la perfusion du placenta 5. Des capteurs de pH, de pression, de température, d'oxygène, de CO₂, ou de composés chimiques ou biologiques prédéterminés, tels que l'acide lactique et/ou le glucose, peuvent être agencés par exemple dans la cavité 2, dans le réservoir de solution maternelle 15 et/ou dans le réservoir de solution fœtale 16, ou de manière plus générale présentés à une partie de la solution maternelle m et/ou de la solution fœtale f.

Les différents capteurs peuvent permettre d'évaluer les variations de composés test C et/ou de composés métabolites lors de la perfusion des faces maternelle et fœtale.

En outre, les mesures du ou des capteurs peuvent également être la ou les entrées d'un système d'asservissement continu d'un paramètre de la solution maternelle m et/ou de la solution fœtale f. Préférentiellement, la concentration d'un composé de la solution maternelle et/ou fœtale, et/ou la température de la solution maternelle et/ou fœtale peuvent être asservies. Le système 1 peut par exemple comprendre un régulateur, mis en œuvre par une mémoire et un calculateur. La mémoire peut comprendre une valeur consigne d'un paramètre de la solution maternelle m et/ou de la solution fœtale f, par exemple la concentration en glucose de la solution maternelle m et/ou de la solution fœtale ou la concentration en composé C de la solution maternelle et/ou de la solution fœtale. Le système peut comprendre un actionneur, commandé par le régulateur. L'actionneur peut par exemple être un contrôleur de pression, un contrôleur de débit ou un système d'injection d'un composé dans la solution maternelle m ou dans la solution fœtale f. Ainsi, il est possible de contrôler l'évolution d'un ou plusieurs paramètres du système 1 de manière à reproduire en partie l'environnement physiologique du placenta, et ainsi permettre un fonctionnement physiologique *ex-vivo* du placenta plus long. Il est également possible de reproduire une partie des conditions pathologiques ou caractéristiques d'une pathologie de la grossesse du placenta 5.

En référence à la figure 5, le diamètre de la cavité 2 peut être inférieur au diamètre de la chambre de réception 3, celle-ci restant bien sûr en regard de l'orifice 9. Ainsi, il est possible de réduire le volume de solution maternelle m. La réduction du volume de solution maternelle m permet d'augmenter la précision de mesure d'un composé métabolite Cm. En effet, si le placenta 5 produit un composé métabolite Cm passant dans la solution maternelle, la diminution du volume de solution maternelle m permet de diminuer la dilution du composé métabolite Cm. La diminution du volume de solution maternelle m peut également permettre de raccourcir la latence de mesure d'un composé test C en solution et/ou de faciliter le maintien et le contrôle de la température. Enfin, la réduction du volume de solution maternelle m permet également de diminuer la quantité du composé test. Typiquement, le volume de la cavité 2 peut être sensiblement égal à 200 mL, de manière préférée, ce volume est inférieur, par exemple à 100mL, de manière encore plus préférée inférieur à 50 mL.

Le système 1 peut également comprendre un capot permettant de fermer la chambre de réception 3. Le capot peut présenter un diamètre adapté à emboîter le capot dans la chambre de réception 3. Ainsi, le transfert thermique entre la cavité 2 et l'environnement extérieur peut être réduit. Le capot peut également présenter des trous ou des ouvertures, permettant de faire passer les tubulures reliées aux cathéters 8 à l'extérieur de la chambre de réception 3.

Le système 1 peut également comprendre un chauffage et un thermostat, configurés pour réguler la température à l'intérieur de la chambre de réception 3 et/ou la température de la solution maternelle m et/ou la température de la solution fœtale f.

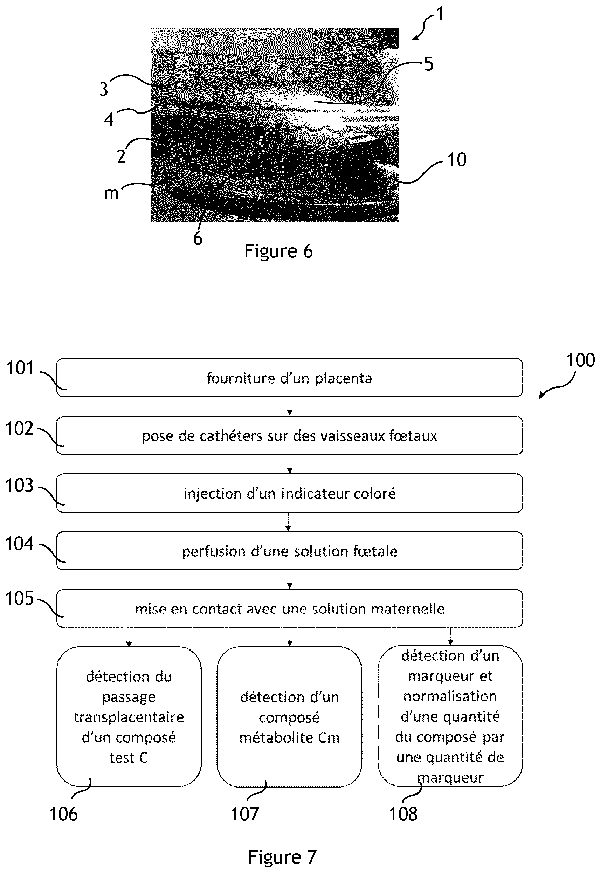

La figure 6 est une photographie illustrant un système 1. Le placenta 5 est supporté par la paroi 4. Une partie des villosités placentaires, ou la surface de la face maternelle 6 correspondant à ces villosités placentaires, est en contact avec la solution maternelle m en écoulement dans la cavité 2. Ainsi, les différents composés de la solution maternelle peuvent passer dans le placenta 5 vers la circulation ou l'écoulement de la solution fœtale f.

### Protocole expérimental

En référence à la figure 7, un objet de l'invention est une méthode 100 d'étude du passage transplacentaire d'un composé test C ou de la métabolisation d'un composé test C en un composé métabolite Cm. Ladite méthode ne comprenant pas l'utilisation d'embryon humain.

### Fourniture d'un placenta

Lors d'une première étape 101, un placenta 5 dont la structure est non consolidée, en particulier âgé d'un mois et demi à quatre mois est fourni. Un tel placenta est issu d'une interruption volontaire de grossesse (IVG) ou d'une interruption médicale de grossesse. Le placenta est considéré dans de tels cas comme un déchet biologique. Le placenta 5 est alors déposé face maternelle vers le bas dans la chambre de réception 3 sur la paroi 4, et en particulier au moins sur le ou l'un des orifices 9.

Dans un mode de réalisation particulier, le placenta 5 dont la structure est non consolidée est un placenta prélevé sur un grand singe, par exemple un grand singe non humain.

### Connexion des cathéters et agencement du placenta

Lors d'une étape 102, des cathéters 8 sont reliés fluidiquement à des vaisseaux correspondant à la circulation fœtale du placenta 5, sur la face fœtale 7. En particulier, on peut relier lors de l'étape 102 un cathéter d'injection 81 dans une artère fœtale et un cathéter de collecte 82 dans une veine fœtale. Ainsi, les cathéters 8 peuvent relier fluidiquement le réseau ou une partie du réseau correspondant à la circulation fœtale du placenta 5 avec un réseau fluidique extérieur, comprenant par exemple un réservoir de solution fœtale 16. Il est ainsi possible d'entraîner un écoulement de solution fœtale f dans une partie des vaisseaux du placenta correspondant à la circulation fœtale.

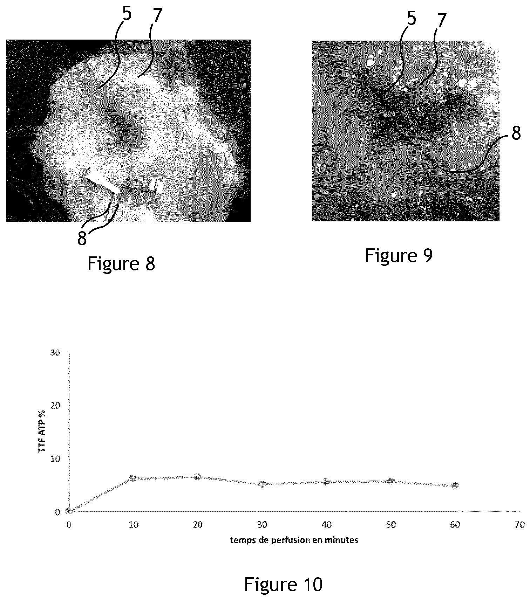

La figure 8 est une photographie en vue de dessus du placenta 5, illustrant la pose de deux cathéters 8 dans le placenta 5, et plus particulièrement sur la face fœtale 7 du placenta 5. Préférentiellement, une partie rigide d'un cathéter peut être insérée au travers de la face fœtale 7 de manière à être relié fluidiquement à un vaisseau. Une fois le cathéter 8 relié à un vaisseau, la partie rigide du cathéter peut par exemple être attachée par compression, ou *clampée,* sur le tissu du placenta 5.

En référence à la figure 9, lors d'une étape 103 de la méthode 100, on peut injecter une solution fœtale de marquage f' comprenant un indicateur coloré dans les vaisseaux fœtaux du placenta. Il est ainsi possible de définir visuellement la zone du placenta 5 irriguée par la solution fœtale de marquage f'. Cette zone est illustrée par une délimitation en pointillés dans la figure 9. Ainsi, il est possible de faire correspondre la position de la partie du placenta 5 irriguée par une solution fœtale f, pour une connexion de cathéters 8 mise en œuvre lors de l'étape 102, avec la position de l'orifice 9 de la paroi 4. Cette correspondance permet d'irriguer la même partie du placenta 5 en solution maternelle m, par trempage des villosités dans la solution maternelle sans cathéter, et en solution fœtale f par la cathétérisation d'une artère et d'une veine fœtale, de manière à permettre le passage transplacentaire d'un composé test C compris dans la solution maternelle m vers la solution fœtale f, ou inversement, ou la métabolisation du composé test C en composé métabolite Cm et son passage dans la solution fœtale f, ou inversement.

### Perfusion d'une solution fœtale

Lors d'une étape 104 de la méthode 100, on perfuse la solution fœtale f dans des vaisseaux de la face fœtale 7, et préférentiellement par injection de la solution fœtale f dans l'artère fœtale via le cathéter d'injection 81 et par récupération de la solution fœtale f via le cathéter de collecte 82 à partir de la veine fœtale, de manière à irriguer des villosités placentaires présentées par la face maternelle 6 du placenta.

La solution fœtale f peut être injectée à un débit compris entre 10 µL.min⁻¹ et 800 µL.min⁻¹, préférentiellement entre 50 µL.min⁻¹ et 300 µL.min⁻¹ et encore plus préférentiellement entre 100 µL.min⁻¹ et 150 µL.min⁻¹. Ainsi, il est possible de mimer le débit physiologique de la circulation sanguine fœtale d'un placenta âgé d'un mois et demi à 4 mois. La ou les gammes de débits représentatives d'une pathologie, par exemple d'une hypoperfusion fœtale ou d'une hyperperfusion fœtale évoluent de manière connue avec l'âge du placenta. Pour l'ensemble des villosités placentaires, le débit de sang fœtal d'une population de fœtus et son évolution en fonction des semaines de gestation peuvent par exemple être décrits par le tableau 1 suivant :

**Tableau 1**

| **gestation (semaines)** | Percentile | | | | | | | | |
|---|---|---|---|---|---|---|---|---|---|
| | **2,5** | **5** | **10** | **25** | **50** | **75** | **90** | **95** | **97,5** |
| | débit (mL/min) | | | | | | | | |
| **11** | 0,50 | 0,60 | 0,70 | 0,90 | 1,20 | 1,60 | 2,10 | 2,50 | 2,80 |
| **12** | 0,80 | 0,90 | 1,00 | 1,40 | 1,80 | 2,40 | 3,00 | 3,50 | 3,90 |
| **13** | 1,20 | 1,40 | 1,60 | 2,10 | 2,70 | 3,50 | 4,50 | 5,10 | 5,70 |
| **14** | 1,90 | 2,10 | 2,50 | 3,10 | 4,00 | 5,10 | 6,40 | 7,30 | 8,10 |
| **15** | 2,80 | 3,10 | 3,60 | 4,50 | 5,70 | 7,30 | 8,90 | 10,10 | 11,20 |
| **16** | 4,00 | 4,50 | 5,10 | 6,30 | 8,00 | 10,10 | 12,30 | 13,80 | 15,30 |
| **17** | 5,60 | 6,20 | 7,10 | 8,70 | 10,90 | 13,70 | 16,60 | 18,70 | 20,60 |
| **18** | 7,60 | 8,50 | 9,60 | 11,80 | 14,70 | 18,20 | 22,10 | 24,70 | 27,30 |
| **19** | 10,20 | 11,30 | 12,80 | 15,60 | 19,40 | 24,00 | 29,00 | 32,40 | 35,60 |
| **20** | 13,40 | 14,80 | 16,70 | 20,40 | 25,30 | 31,20 | 37,50 | 41,90 | 46,00 |

Le calcul d'un débit physiologique peut par exemple être calculé en fonction de la proportion du placenta irrigué par la solution fœtale par rapport à la totalité de la face maternelle du placenta, au regard des débits donnés par le tableau 1. Il est également possible de reproduire une partie des conditions pathologiques du placenta 5 en contrôlant des débits spécifiques de solution fœtale f dans le placenta 5. Par exemple, on peut mimer un cas d'hyperperfusion fœtale dans le placenta 5. On peut également mimer un cas d'hypoperfusion fœtale dans le placenta 5. L'homme du métier sait, en fonction de l'âge du placenta choisir des débits correspondant à une hyperperfusion ou une hypoperfusion.

### Mise en contact du placenta avec un composé test

Le passage d'un composé test C de la solution maternelle vers la solution fœtale peut par exemple être étudié, comme cela est décrit dans la suite des étapes d'un exemple de procédé selon l'invention présenté ici. Lors d'une étape 105 de la méthode 100, on met en contact au moins une partie des villosités placentaires irriguées lors de l'étape 104 par une solution fœtale f avec une solution maternelle m comprenant au moins un composé test C. La solution maternelle m peut être statique dans la cavité 2 ou en écoulement dans la cavité 2. Le contrôle d'un écoulement de solution maternelle m dans la cavité 2 permet de renouveler localement la concentration en composé test C au voisinage de la face maternelle 6. En effet, les inventeurs ont découvert que la mise en contact de la face maternelle 6 avec la solution maternelle m permettait le passage du composé test C de la solution maternelle m dans le placenta 5. Lors de ce passage, la concentration du composé C dans la solution maternelle m vers le placenta entraîne une diminution de la concentration de composé C dans la solution maternelle m au voisinage du placenta. L'écoulement de la solution maternelle m permet de favoriser le transport de composé C au voisinage de la face maternelle 6.

Le débit de solution maternelle dans la cavité 2 peut par exemple être compris entre 1 mL.min⁻¹ et 20 mL.min⁻¹, et préférentiellement entre 5 et 10 mL.min⁻¹. Ainsi, il est possible de transporter un composé test C au voisinage de la face maternelle 6 dans des quantités qui permettent de mimer le transport du composé test C par la circulation sanguine maternelle lors de la grossesse.

Dans un mode de réalisation particulier, il est possible de simuler une hypoperfusion du placenta 5 en choisissant un débit plus faible que le débit permettant de traduire les conditions physiologiques normales d'apport en un composé test C. Préférentiellement, on peut contrôler un débit de solution maternelle m compris entre 1 mL.min⁻¹ et 5 mL.min⁻¹ de manière à simuler l'hypoperfusion. Dans un mode de réalisation particulier, il est possible de simuler une hyperperfusion du placenta 5 en choisissant un débit plus élevé que le débit permettant de traduire les conditions physiologiques normales d'apport en un composé test C. Ainsi, préférentiellement, on peut contrôler un débit de solution maternelle m compris entre 10 mL.min⁻¹ et 20 mL.min⁻¹ de manière à simuler l'hyperperfusion.

La ou les gammes de débits représentatifs d'une pathologie, par exemple d'une hypoperfusion fœtale ou d'une hyperperfusion maternelle évoluent de manière connue avec l'âge du placenta.

### Détection du passage d'un composé test

Lors d'une étape 106 et/ou 107 de la méthode 100, on détecte le passage d'un composé test C de la solution maternelle au travers du le placenta 5.

Lors de l'étape 106, on détecte le passage transplacentaire d'un composé test C. La détection du composé C peut être mise en œuvre par plusieurs moyens. On peut par exemple prélever puis analyser un échantillon de solution maternelle m dans la cavité 2 ou depuis un point de circulation fluidique en aval de la cavité 2 quand la circulation de solution maternelle m est ouverte. Par exemple, l'analyse peut être mise en œuvre par chromatographie en phase liquide. Dans le cas d'une circulation de solution maternelle m en boucle fermée, il est possible de prélever la solution maternelle m à n'importe quel point de la boucle. Un ou plusieurs capteurs chimiques ou biologiques peuvent également être arrangés dans la cavité 2 et/ou dans le réservoir de solution maternelle 15. La détection du composé test C dans la solution maternelle m peut traduire non seulement le passage du composé test C de la solution maternelle m vers le placenta 5 (disparition du composé test C), mais également la réintroduction du composé test C dans la solution maternelle m depuis la solution fœtale *via* le placenta (introduction du composé test C, par exemple rendue possible par l'introduction antérieure du composé test C de la solution maternelle m vers la solution fœtale f).

Le passage du composé test C peut être détecté par prélèvement et analyse de la solution fœtale f, ou par détection directe dans le système 1 du composé test C par un ou plusieurs capteurs chimiques ou biologiques arrangés dans la circulation de solution fœtale f, par exemple dans le réservoir de solution fœtale f.

Le passage d'un composé test C peut également être étudié en détectant un composé métabolite Cm, issu de la métabolisation du composé test C dans le placenta 5. Lors d'une étape 107, on détecte un composé métabolite Cm dans le système 1. La détection du composé métabolite Cm peut être réalisée par prélèvement de la solution fœtale f puis par analyse et/ou par détection directe par des capteurs chimiques et/ou biologiques arrangés dans la circulation de la solution fœtale f et/ou de la solution maternelle m.

Lors d'une étape 108, on détecte un composé marqueur dans la solution maternelle m et/ou dans la solution fœtale f. Ainsi, il est possible de valider la possibilité d'un échange transplacentaire après la mise en œuvre des étapes 101 à 105. On peut préférentiellement normaliser la quantité de composé test C mesurée et/ou la quantité de composé métabolite Cm mesurée par la concentration de composé marqueur mesurée. Ainsi, il est possible d'étudier le passage d'un composé test C ou l'apparition d'un composé métabolite Cm en diminuant l'influence des caractéristiques propres au placenta 5 lors de la mesure, et d'augmenter la reproductibilité des résultats d'étude de passage transplacentaire. Le composé marqueur présente un taux de passage transplacentaire prédéterminé. Ce taux peut être élevé, par exemple compris entre 80% et 100%, préférentiellement entre 90% et 100% et encore plus préférentiellement entre 95% et 100%, ou particulièrement faible, par exemple compris entre 0% et 20%, préférentiellement compris entre 0% et 10% et encore plus préférentiellement compris entre 0% et 5%.

Dans un mode de réalisation particulier, les composés C et/ou Cm ou les composés marqueurs peuvent également être quantifiés, détectés et/ou localisés dans le placenta ou ses sous-structures.

Les mêmes étapes peuvent être appliquées à partir de l'étape 105 concernant, mutatis mutandis, l'étude du passage d'un composé test C de la solution fœtale vers la solution maternelle.

Un composé marqueur de passage transplacentaire peut être choisi parmi au moins l'antipyrine, l'insuline, le dextran, le fuzeon (enfuvirtide) et l'AZT.

Dans un mode de réalisation particulier, la solution maternelle m et/ou la solution fœtale peut comprendre un inhibiteur de transporteur placentaire. Ainsi, il est possible d'étudier l'effet de l'inhibition d'un transporteur placentaire prédéterminé sur le transport transplacentaire du composé test C.

### Résultats

En référence à la figure 10, on peut mesurer la cinétique du taux de transfert de l'antipyrine (TTF ATP). Ainsi, il est possible de mesurer le temps au bout duquel l'évolution de la concentration d'un composé mesuré, en l'occurrence l'antipyrine dans la solution maternelle m, est sensiblement nulle et stationnaire. Ce temps correspond à une situation d'équilibre des concentrations d'antipyrine mesurées dans les circulations de solution maternelle m et de solution fœtale f.

## Revendications

1. Système (1) d'étude du passage ou de la métabolisation transplacentaire *ex-vivo* d'un composé test (C), comprenant :
- une chambre supérieure de réception (3) d'un placenta (5), au moins partiellement définie par une paroi (4) sur laquelle est déposée le placenta (5), et présentant au moins un orifice (9),
- un cathéter d'injection (81) adapté pour injecter une solution fœtale (f) dans l'artère fœtale du placenta et irriguer des villosités placentaires lorsque le placenta est reçu dans la chambre de réception (3),
- un cathéter de collecte (82) adapté pour collecter la solution fœtale (f) depuis la veine fœtale du placenta, lorsque le placenta est reçu dans la chambre de réception,
- des moyens de mise en contact d'au moins une partie des villosités placentaires irriguées par la solution fœtale (f) avec une solution maternelle (m), lorsque le placenta est reçu dans la chambre de réception, ladite solution fœtale (f) et/ou ladite solution maternelle (m) comprenant au moins un composé test (C),
**Caractérisé en ce que** :
- la chambre de réception supérieure (3) est destinée à recevoir un placenta (5) qui ne présente pas de septa intercotylédonaires définissant des cotylédons, et qui est agencé de manière à présenter la face fœtale (7) du placenta (5) vers la partie supérieure du système (1), qui se situe au-dessus de la paroi (4) et la face maternelle (6) en regard de la paroi (4),
- les moyens de mise en contact comprennent une cavité inférieure (2) au moins partiellement définie par la paroi (4) et adaptée à contenir la solution maternelle (m),
- la paroi (4) séparant la cavité inférieure (2) et la chambre supérieure de réception (3) du placenta, de sorte qu'au moins une partie des villosités placentaires (5) du placenta supporté par la paroi (4) dans la chambre supérieure de réception (3) puisse être mise en contact librement par trempage dans la solution maternelle (m) contenue dans la cavité inférieure (2) via l'orifice (9),
- la position de la partie du placenta (5) irriguée par la solution fœtale f, correspondant à la position de l'orifice (9) de la paroi (4),
- la cavité inférieure (2) comprenant une entrée fluidique (10) et une sortie fluidique (11), l'entrée fluidique (10) et la sortie fluidique (11) permettent de commander un écoulement de solution maternelle m dans la cavité inférieure (2),
le système (1) permettant de rétablir, dans un placenta qui ne présente pas de septa intercotylédonaires définissant des cotylédons, une double circulation materno-fœtale.

2. Système selon la revendication 1, dans lequel le diamètre de l'orifice (9) est inférieur à 5 cm et d'au moins 1 cm.

3. Système selon l'une quelconque des revendications 1 ou 2, comprenant une première conduite (12), dans lequel le cathéter d'injection (81) et le cathéter de collecte (82) sont reliés fluidiquement par au moins la première conduite (12), apte à rendre l'écoulement de la solution fœtale f en circuit fermé, le système comprenant une pompe (14) adaptée pour entraîner un écoulement dans ladite première conduite (12).

4. Système selon l'une quelconque des revendications 1 à 3, comprenant une deuxième conduite (13) reliée à la chambre de perfusion, l'entrée fluidique (10) et la sortie fluidique (11) étant reliées fluidiquement par au moins la deuxième conduite (13), apte à rendre l'écoulement de la solution maternelle m en circuit fermé, le système comprenant une pompe (14) adaptée pour entraîner un écoulement dans la deuxième conduite (13).

5. Système selon l'une quelconque des revendications 1 à 4, comprenant un placenta (5) qui ne présente pas de septa intercotylédonaires définissant des cotylédons et qui, quand il est d'origine humaine, est un déchet biologique issu d'une interruption volontaire de grossesse ou d'une interruption médicale de grossesse.

6. Système selon l'une quelconque des revendications 1 à 5, dans lequel le système (1) comprend un élément choisi parmi un capteur de pH, un capteur de température, un capteur d'oxygène, un capteur de pression et un moyen de mesure de la concentration du composé test (C) et/ou d'un composé métabolite (Cm) dont le passage transplacentaire est testé.

7. Système selon l'une quelconque des revendications 1 à 6, comprenant des moyens d'asservissement de la concentration d'un composé de la solution maternelle (m) et/ou de la solution fœtale (f) à une valeur consignée.

8. Système selon l'une quelconque des revendications 1 à 7, comprenant plusieurs parois (4) interchangeables avec des orifices (9) de tailles différentes, centrés ou décentrés, de manière à adapter au mieux le système au placenta étudié.

9. Système selon l'une quelconque des revendications 1 à 8, dans lequel la cavité (2) présente une ouverture configurée pour permettre un prélèvement de la solution maternelle.

10. Méthode (100) *ex-vivo* d'étude du passage transplacentaire d'un composé test (C) ou de la métabolisation d'un composé test (C) en un composé métabolite (Cm), utilisant le système selon l'une quelconque des revendications 1 à 9, comprenant les étapes suivantes :
a) fourniture d'un placenta (5) qui ne présente pas de septa intercotylédonaires définissant des cotylédons, ledit placenta, quand il est d'origine humaine, étant un déchet biologique issu d'une interruption volontaire de grossesse ou d'une interruption médicale de grossesse présentant une face maternelle (6) et une face fœtale (7) et comprenant une artère fœtale et une veine fœtale,
b) pose d'un cathéter d'injection (81) sur une artère fœtale et pose d'un cathéter de collecte (82) sur une veine fœtale,
c) perfusion du placenta (5) par injection via le cathéter d'injection (81) d'une solution fœtale (f) dans l'artère fœtale et récupération via un cathéter de collecte (82) de la solution fœtale à partir de la veine fœtale, de manière à irriguer des villosités placentaires présentées par la face maternelle (6) du placenta,
d) mise en contact d'au moins une partie des villosités placentaires irriguées par la solution fœtale (f) et présentées par la face maternelle (6) du placenta avec une solution maternelle, ladite solution fœtale et/ou ladite solution maternelle comprenant au moins un composé test,
e) détection du passage transplacentaire du composé test (C) et/ou de la métabolisation du composé test (C) en composé métabolite (Cm).

11. Méthode selon la revendication 10, comprenant une étape b') après l'étape b) et avant l'étape c), l'étape b') comprenant la perfusion du placenta (5) par injection via un cathéter (81) au niveau de l'artère fœtale d'une solution fœtale (f') comprenant un indicateur coloré permettant d'identifier au moins une villosité placentaire irriguée par ladite solution fœtale (f').

12. Méthode selon l'une quelconque des revendications 10 ou 11, **caractérisée en ce que** la solution fœtale et/ou la solution maternelle contient un composé marqueur, ledit composé marqueur présentant un taux de passage transplacentaire prédéterminé.

13. Méthode selon la revendication 12, dans laquelle le composé marqueur est l'antipyrine.

14. Méthode selon l'une quelconque des revendications 12 ou 13, dans laquelle on normalise la quantité de composé test (C) et/ou la quantité de composé métabolite (Cm) détectée lors de l'étape e) par une quantité de composé marqueur mesurée.

15. Méthode selon l'une quelconque des revendications 10 à 14, dans laquelle la solution maternelle (m) comprend un inhibiteur de transporteur placentaire.

16. Méthode selon l'une quelconque des revendications 10 à 15, dans laquelle la cavité remplie de solution maternelle (m) est alimentée en ladite solution par une entrée fluidique (10) et vidée en ladite solution par une sortie fluidique (11) afin de créer un écoulement de solution maternelle à un débit compris entre 1 mL.min⁻¹ et 20 mL.min⁻¹.

17. Méthode selon l'une quelconque des revendications 10 à 16, dans laquelle le débit de solution fœtale est compris entre 50 µL.min⁻¹ et 300 µL.min⁻¹.

18. Méthode selon l'une quelconque des revendications 10 à 17, dans laquelle la solution maternelle et/ou la solution fœtale comprend un microorganisme ou un fragment de microorganisme choisi au moins parmi une bactérie, un virus, un organisme unicellulaire et une molécule issue d'une bactérie, d'un virus ou d'un organisme unicellulaire.

## Patentansprüche

1. System (1) zur Erforschung der transplazentaren Passage oder der transplazentaren Metabolisierung *ex-vivo* einer Testverbindung (C), umfassend:
- eine obere Aufnahmekammer (3) für eine Plazenta (5), die mindestens teilweise durch eine Wand (4) definiert ist, auf der die Plazenta (5) abgelegt ist, und die mindestens eine Öffnung (9) aufweist,
- einen Injektionskatheter (81), der zum Injizieren einer fetalen Lösung (f) in die fetale Arterie der Plazenta und Durchspülen der Plazentazotten angepasst ist, wenn die Plazenta in der Aufnahmekammer (3) aufgenommen ist,
- einen Sammelkatheter (82), der zum Sammeln der fetalen Lösung (f) aus der fetalen Vene der Plazenta angepasst ist, wenn die Plazenta in der Aufnahmekammer aufgenommen ist,
- Mittel zum In-Kontakt-Bringen mindestens eines Teils der Plazentazotten, die mit der fetalen Lösung (f) durchgespült sind, mit einer mütterlichen Lösung (m), wenn die Plazenta in der Aufnahmekammer aufgenommen ist, die fetale Lösung (f) und/oder die mütterliche Lösung (m) umfassend mindestens eine Testverbindung (C),
**dadurch gekennzeichnet, dass:**
- die obere Aufnahmekammer (3) vorgesehen ist, eine Plazenta (5) aufzunehmen, die keine interkotyledonären Septen aufweist, die Kotyledonen definieren, und die angeordnet ist, um die fetale Seite (7) der Plazenta (5) zu dem oberen Teil des Systems (1) hin aufzuweisen, die oberhalb der Wand (4) liegt, und wobei die mütterliche Seite (6) der Wand (4) zugewandt ist,
- die Mittel zum In-Kontakt-Bringen einen unteren Hohlraum (2), der mindestens teilweise durch die Wand (4) definiert und angepasst ist, die Mutterlösung (m) zu enthalten, umfassen,
- die Wand (4), die den unteren Hohlraum (2) und die obere Aufnahmekammer (3) der Plazenta trennt, derart, dass mindestens ein Teil der Plazentazotten (5) der Plazenta, die von der Wand (4) in der oberen Aufnahmekammer (3) getragen wird, durch Eintauchen in die mütterliche Lösung (m), die in dem unteren Hohlraum (2) enthalten ist, über die Öffnung (9) frei in Kontakt gebracht werden kann,
- die Position des Teils der Plazenta (5), der mit der fetalen Lösung f durchgespült wird, der Position der Öffnung (9) der Wand (4) entspricht,
- der untere Hohlraum (2) einen Fluideinlass (10) und einen Fluidauslass (11) umfasst, wobei der Fluideinlass (10) und der Fluidauslass (11) es ermöglichen, ein Strömen der mütterlichen Lösung m in dem unteren Hohlraum (2) zu steuern,
wobei das System (1) ermöglicht, in einer Plazenta, die keine interkotyledonären Septen aufweist, die Kotyledonen definieren, einen doppelten materno-fetalen Kreislauf wiederherzustellen.

2. System nach Anspruch 1, wobei der Durchmesser der Öffnung (9) kleiner als 5 cm ist und mindestens 1 cm beträgt.

3. System nach einem der Ansprüche 1 oder 2, umfassend eine erste Leitung (12), wobei der Injektionskatheter (81) und der Sammelkatheter (82) durch mindestens die erste Leitung (12) fluidisch verbunden sind, die geeignet ist, das Strömen der fetalen Lösung f in einem geschlossenen Kreislauf zu realisieren, das System umfassend eine Pumpe (14), die zum Antreiben eines Strömens in der ersten Leitung (12) angepasst ist.

4. System nach einem der Ansprüche 1 bis 3, umfassend eine zweite Leitung (13), die mit der Perfusionskammer verbunden ist, wobei der Fluideinlass (10) und der Fluidauslass (11) durch mindestens die zweite Leitung (13) fluidisch verbunden sind, die geeignet ist, das Strömen der mütterlichen Lösung m in einem geschlossenen Kreislauf zu realisieren, das System umfassend eine Pumpe (14), die zum Antreiben eines Strömens in der zweiten Leitung (13) angepasst ist.

5. System nach einem der Ansprüche 1 bis 4, umfassend eine Plazenta (5), die keine interkotyledonären Septen aufweist, die Kotyledonen definieren, und die, wenn sie menschlichen Ursprungs ist, ein biologischer Abfall aus einem freiwilligen Schwangerschaftsabbruch oder einem medizinischen Schwangerschaftsabbruch stammt.

6. System nach einem der Ansprüche 1 bis 5, wobei das System (1) ein Element umfasst, das ausgewählt ist aus einem pH-Wert-Sensor, einem Temperatursensor, einem Sauerstoffsensor, einem Drucksensor und einem Mittel für die Messung der Konzentration der Testverbindung (C) und/oder einer Metabolitverbindung (Cm), deren transplazentare Passage getestet wird.

7. System nach einem der Ansprüche 1 bis 6, umfassend Mittel für die Regelung der Konzentration einer Verbindung der mütterlichen Lösung (m) und/oder der fötalen Lösung (f) auf einen Sollwert.

8. System nach einem der Ansprüche 1 bis 7, umfassend mehrere austauschbare Wände (4) mit Öffnungen (9) unterschiedlicher Größe, zentriert oder dezentriert, um das System bestmöglich an die untersuchte Plazenta anzupassen.

9. System nach einem der Ansprüche 1 bis 8, wobei der Hohlraum (2) eine Apertur aufweist, die zum Ermöglichen einer Entnahme der mütterlichen Lösung konfiguriert ist.

10. *Ex-vivo*-Verfahren (100) zur Erforschung der transplazentaren Passage einer Testverbindung (C) oder der Metabolisierung einer Testverbindung (C) zu einer Metabolitverbindung (Cm) unter Verwendung des Systems nach einem der Ansprüche 1 bis 9, umfassend die folgenden Schritte:
a) Bereitstellen einer Plazenta (5), die keine interkotyledonären Septen aufweist, die Kotyledonen definieren, wobei die Plazenta, wenn sie menschlichen Ursprungs ist, ein biologischer Abfall ist, der aus einem freiwilligen Schwangerschaftsabbruch oder einem medizinischen Schwangerschaftsabbruch stammt, die eine mütterliche Seite (6) und eine fötale Seite (7) aufweist und umfassend eine fötale Arterie und eine fötale Vene,
b) Anlegen eines Injektionskatheters (81) an eine fetale Arterie und Anlegen eines Sammelkatheters (82) an eine fetalen Vene,
c) Infundieren der Plazenta (5) durch Injektion, über den Injektionskatheter (81), einer fetalen Lösung (f) in die fetale Arterie und Rückgewinnung, über einen Sammelkatheter (82), der fetalen Lösung aus der fetalen Vene, um Plazentazotten durchzuspülen, die sich auf der mütterlichen Seite (6) der Plazenta darstellen,
d) Inkontaktbringen mindestens eines Teils der Plazentazotten, die mit der fetalen Lösung (f) durchgespült werden und die sich auf der mütterlichen Seite (6) der Plazenta darstellen, mit einer mütterlichen Lösung, die fetale Lösung und/oder die mütterliche Lösung umfassend mindestens eine Testverbindung,
e) Erfassen der transplazentaren Passage der Testverbindung (C) und/oder der Metabolisierung der Testverbindung (C) zu der Metabolitverbindung (Cm).

11. Verfahren nach Anspruch 10, umfassend einen Schritt b') nach Schritt b) und vor Schritt c), Schritt b') umfassend das Infundieren der Plazenta (5) durch Injektion, über einen Katheter (81), an der fetalen Arterie, einer fetalen Lösung (f'), umfassend einen Farbindikator, der es ermöglicht, mindestens eine Plazentazotte zu identifizieren, die mit der fetalen Lösung (f') durchgespült wird.

12. Verfahren nach einem der Ansprüche 10 oder 11,
**dadurch gekennzeichnet, dass** die fetale Lösung und/oder die mütterliche Lösung eine Markerverbindung enthält, wobei die Markerverbindung eine vorbestimmte transplazentare Passagerate aufweist.

13. Verfahren nach Anspruch 12, wobei die Markerverbindung Antipyrin ist.

14. Verfahren nach einem der Ansprüche 12 oder 13, wobei die Menge an Testverbindung (C) und/oder Menge an Metabolitverbindung (Cm), die während des Schritts e) erfasst wird, durch eine gemessene Menge an Markerverbindung eingestellt wird.

15. Verfahren nach einem der Ansprüche 10 bis 14, wobei die mütterliche Lösung (m) einen plazentaren Transporterinhibitor umfasst.

16. Verfahren nach einem der Ansprüche 10 bis 15, wobei der Hohlraum, der mit mütterlicher Lösung (m) gefüllt wird, mit der Lösung durch einen Fluideinlass (10) gespeist wird und von der Lösung über einen Fluidauslass (11) entleert wird, um ein Strömen der mütterlichen Lösung mit einer Durchflussrate, die zwischen 1 ml/min⁻¹ und 20 ml/min⁻¹ liegt, zu erzeugen.

17. Verfahren nach einem der Ansprüche 10 bis 16, wobei die Durchflussrate der fötalen Lösung zwischen 50 µl/min⁻¹ und 300 µl/min⁻¹ liegt.

18. Verfahren nach einem der Ansprüche 10 bis 17, wobei die mütterliche Lösung und/oder die fötale Lösung einen Mikroorganismus oder ein Fragment eines Mikroorganismus umfasst, ausgewählt mindestens aus einem Bakterium, einem Virus, einem einzelligen Organismus und einem Molekül, das von einem Bakterium, einem Virus oder einem einzelligen Organismus stammt.

## Claims

1. System (1) for studying the *ex vivo* transplacental passage or metabolization of a test compound (C), the system comprising:
- an upper chamber (3) for receiving a placenta (5), the chamber being at least partially defined by a wall (4) on which the placenta (5) is placed and which has at least one orifice (9),
- an injection catheter (81) suitable for injecting a fetal solution (f) into the fetal artery of the placenta and for irrigating placental villi with the placenta received in the receiving chamber (3),
- a collection catheter (82) suitable for collecting the fetal solution (f) from the fetal vein of the placenta with the placenta received in the receiving chamber,
- means for bringing at least some of the placental villi irrigated with the fetal solution (f) into contact with a maternal solution (m) with the placenta received in the receiving chamber, said fetal solution (f) and/or said maternal solution (m) comprising at least one test compound (C),
**characterized in that**:
- the upper receiving chamber (3) is intended to receive a placenta (5) which has no cotyledon-defining intercotyledonary septa and which is arranged so as to have the fetal side (7) of the placenta (5) toward the upper part of the system (1) which is located above the wall (4) and to have the maternal side (6) facing the wall (4),
- the contacting means comprise a lower cavity (2) at least partially defined by the wall (4) and suitable for containing the maternal solution (m),
- the wall (4) separates the lower cavity (2) and the upper chamber (3) for receiving the placenta, such that at least some the placental villi (5) of the placenta which is supported by the wall (4) in the upper receiving chamber (3) can freely be brought into contact by being immersed in the maternal solution (m) contained in the lower cavity (2) via the orifice (9),
- the position of the part of the placenta (5) which is irrigated with the fetal fluid f corresponds to the position of the orifice (9) of the wall (4),
- the lower cavity (2) comprises a fluid inlet (10) and a fluid outlet (11), the fluid inlet (10) and the fluid outlet (11) allowing a flow of maternal solution m into the lower cavity (2) to be controlled,
the system (1) allowing dual maternofetal circulation to be restored in a placenta which has no cotyledon-defining intercotyledonary septa.

2. System according to claim 1, wherein the diameter of the orifice (9) is less than 5 cm and at least 1 cm.

3. System according to either of claims 1 or 2, comprising a first line (12), wherein the injection catheter (81) and the collection catheter (82) are fluidically connected by at least the first line (12) which is able to cause the fetal solution f to flow in a closed loop, the system comprising a pump (14) suitable for driving a flow in said first line (12).

4. System according to any of claims 1 to 3, comprising a second line (13) connected to the perfusion chamber, the fluid inlet (10) and the fluid outlet (11) being fluidically connected by at least the second line (13) which is able to cause the maternal solution m to flow in a closed loop, the system comprising a pump (14) suitable for driving a flow in the second line (13).

5. System according to any of claims 1 to 4, comprising a placenta (5) which has no cotyledon-defining intercotyledonary septa and which, when of human origin, is biological waste resulting from a voluntary termination of pregnancy or a medically indicated termination of pregnancy.

6. System according to any of claims 1 to 5, wherein the system (1) comprises an element selected from a pH sensor, a temperature sensor, an oxygen sensor, a pressure sensor, and a means for measuring the concentration of the test compound (C) and/or a metabolite compound (Cm) of which the transplacental passage is being tested.

7. System according to any of claims 1 to 6, comprising means for controlling the concentration of a compound in the maternal solution (m) and/or the fetal solution (f) to a setpoint value.

8. System according to any of claims 1 to 7, comprising a plurality of interchangeable walls (4) with different sizes of centered of eccentric orifices (9), so as to best adapt the system to the placenta being studied.

9. System according to any of claims 1 to 8, wherein the cavity (2) has an opening configured to allow the maternal solution to be sampled.

10. *Ex vivo* method (100) for studying, using the system according to any of claims 1 to 9, the transplacental passage of a test compound (C) or the metabolization of a test compound (C) into a metabolite compound (Cm), the method comprising the following steps:
a) providing a placenta (5) which has no cotyledon-defining intercotyledonary septa, said placenta, when of human origin, being biological waste resulting from a voluntary termination of pregnancy or a medically indicated termination of pregnancy, having a maternal side (6) and a fetal side (7), and comprising a fetal artery and a fetal vein,
b) positioning an injection catheter (81) in a fetal artery and positioning a collection catheter (82) in a fetal vein,
c) perfusing the placenta (5) by injecting via the injection catheter (81) a fetal solution (f) into the fetal artery and by recovering via a collection catheter (82) the fetal solution from the fetal vein, so as to irrigate placental villi of the maternal side (6) of the placenta,
d) bringing at least some of the placental villi of the maternal side (6) of the placenta that are irrigated with the fetal solution (f) into contact with a maternal solution, said fetal solution and/or said maternal solution comprising at least one test compound,
e) detecting the transplacental passage of the test compound (C) and/or the metabolization of the test compound (C) into a metabolite compound (Cm).

11. Method according to claim 10, comprising a step b') after the step b) and before the step c), the step b') comprising perfusing the placenta (5) by injecting via a catheter (81) at the fetal artery a fetal solution (f') comprising a colored indicator allowing at least one placental villus irrigated with said fetal solution (f') to be identified.

12. Method according to either of claims 10 or 11, **characterized in that** the fetal solution and/or the maternal solution contains a marker compound, said marker compound having a predetermined of transplacental passage rate.

13. Method according to claim 12, wherein the marker compound is antipyrine.

14. Method according to either of claims 12 or 13, wherein the amount of test compound (C) and/or the amount of metabolite compound (Cm) detected in the step e) is normalized by a measured amount of marker compound.

15. Method according to any of claims 10 to 14, wherein the maternal solution (m) comprises a placental transporter inhibitor.

16. Method according to any of claims 10 to 15, wherein the cavity filled with maternal solution (m) is fed with said solution via a fluid inlet (10) and drained of said solution via a fluid outlet (11) to create a maternal solution flow at a flow rate of between 1 mL.min⁻¹ and 20 mL.min⁻¹.

17. Method according to any of claims 10 to 16, wherein the fetal solution flow rate is between 50 µL.min⁻¹ and 300 µL.min⁻¹.

18. Method according to any of claims 10 to 17, wherein the maternal solution and/or the fetal solution comprises a microorganism or a microorganism fragment selected at least from a bacterium, a virus, a unicellular organism, and a molecule derived from a bacterium, a virus, or a unicellular organism.
